Europäisches Patentamt

European Patent Office

Office européen des brevets

(19)

(11) Publication number: **0 140 492**
**B1**

(12) EUROPEAN PATENT SPECIFICATION

(45) Date of publication of patent specification: **11.01.89**

(51) Int. Cl.⁴: **C 07 D 417/12, A 61 K 31/54**

(21) Application number: **84305324.0**

(22) Date of filing: **06.08.84**

(54) L-arginine isoxicamate.

(30) Priority: **28.09.83 US 536481**

(43) Date of publication of application:
**08.05.85 Bulletin 85/19**

(45) Publication of the grant of the patent:
**11.01.89 Bulletin 89/02**

(84) Designated Contracting States:
**AT BE CH DE FR GB IT LI LU NL SE**

(56) References cited:
**EP-A-0 066 458**

**"BURGERS'S MEDICINAL CHEMISTRY", 4.ed.,
Part I, chapter 2, pp. 79/080**
**"JOURNAL OF PHARMACEUTICAL SCIENCES,
January 1977/1, vol. 66, no. 1, pp. 1-19**

(73) Proprietor: **WARNER-LAMBERT COMPANY
201 Tabor Road
Morris Plains New Jersey 07950 (US)**

(72) Inventor: **Chafetz, Lester
144 Grant Avenue
New Providence, NJ 07974 (US)**
Inventor: **Hong, Wen-Hai
10 Sylvan Drive
Pine Brook, NJ 07058 (US)**

(74) Representative: **Jones, Michael Raymond et al
HASELTINE LAKE & CO. Hazlitt House 28
Southampton Buildings Chancery Lane
London WC2A 1AT (GB)**

**Description**

Isoxicam, 4 - hydroxy - 2 - methyl - N - (5 - methyl - 3 - isoxazolyl) - 2$H$ - 1,2 - benzothiazine - 3 - carboxamide - 1,1 - dioxide, is a nonsteroidal anti-inflammatory drug described in U.S. Patent 3,787,324. The compound is weakly acidic (pKa about 3.65) and practically insoluble in water and alcohol. Because of the potential for incomplete or inconsistent bioavailability that often accompanies low aqueous solubility, isoxicam has been formulated in capsule dosage forms using drug that has been micronized or precipitated in microcrystalline form.

The N-methylglucamine salts of oxicam derivatives have been described in European Published Application No. 0002482 and in U.S. Serial Number 488,922 filed on 27th April 1983. These salts were prepared to enhance the solubility and stability of aqueous solutions of the oxicam derivatives.

Water-soluble lysine and arginine salts of piroxicam have been described as especially useful for treating arthritis by parenteral administration in European Published Application No. 0066458.

According to a first aspect of the present invention there is provided a compound having the name L'arginine osoxicamate. This is a novel salt of isoxicam and exhibits surprisingly greater bioavailability when administered to mammals.

The L-arginine isoxicamate may have a 1:1 stoichiometry. In this case, the compound may be in an anhydrous, crystalline state.

According to a second aspect of the present invention, there is provided a pharmaceutical composition, suitable for use as an anti-inflammatory agent or as an analgesic, which comprises an effective amount of L-arginine isoxicamate in admixture with a pharmaceutically acceptable carrier.

According to a third aspect of the present invention provides a process for preparing L-arginine isoxicamate, which process comprises heating L-arginine and isoxicam together in a solvent. The solvent is preferably aqueous methanol, and the heating is conveniently carried out at the boiling point of the solvent.

According to a fourth aspect of the present invention, there is provided a use of L-arginine isoxicamate, optionally in oral dosage form, as an anti-inflammatory agent.

Isoxicam, its alkali metal, alkaline earth and amine salts, including their method of manufacture and therapeutic properties have been fully described in U.S. Patent 3,787,324.

L-arginine isoxicamate may be prepared by heating the suspended reactants, isoxicam and L-arginine, in aqueuos methanol at the boiling point of the solvent. L-arginine isoxicamate, when prepared by this method, is an ahydrous, crystalline salt of 1:1 stoichiometry. It is a fine yellow crystalline powder with a water solubility of 19 mg per ml; a saturated solution exhibiting a pH of about 8.8.

In order to use the compound of the invention, it may be formulated into oral dosage forms such as capsules, tablets, or syrups by blending with an appropriate inert pharmaceutical carrier such as cellulose, lactose, or simple syrup by methods well known to the pharmaceutical art. For injectionable dosage forms, it may be formulated with vehicles such as water. The compound of the invention may also be formulated in topical preparations.

The L-arginine salt of isoxicam exhibits greater and more consistent bioavailability when orally administered than other known forms of isoxicam. Thus, for example, when compared to micronized isoxicam, L-arginine isoxicamate affords a significant improvement in bioavailability on oral administration. Isoxicam plasma concentrations and bioavailability parameters in four dogs after a single 100 mg oral dose of the arginine salt or the free acid are shown in Tables 1 and 2, respectively.

Reference will now be made, by way of example only, to the accompanying drawings in which:

Figure 1 is a line drawing showing the mean plasma time course of four beagle dogs after a single oral dose of 100 mg free acid equivalents of isoxicam;

Figure 2 is a line drawing plotting plasma concentrations versus time profile of isoxicam after a single oral dose of 100 mg free acid equivalents to Beagle Dog 120,642;

Figure 3 is a line drawing plotting plasma concentrations versus time profile of isoxicam after a single oral dose of 100 mg free acid equivalents to Beagle Dog 117,668;

Figure 4 is a line drawing plotting plasma concentration versus time profile of isoxicam after a single oral dose of 100 mg free acid equivalents to beagle Dog 1930; and

Figure 5 is a line drawing plotting plasma concentration versus time profile of isoxicam after a single oral dose of 100 mg free acid equivalents to Beagle Dog 1942.

The numbers following the words "Beagle Dog" in the four immediately preceding paragraphs identify the particular animal being tested.

Following a 100 mg (free acid equivalents) per oral dose of the arginine salt of isoxicam a mean peak plasma concentration of 53.9±4.0 µg/ml was reached at two hours postdose. The range for the individual animals was 51.4 to 63.0 µg/ml with three animals having a peak time of two hours and the final animal at eight hours. These plasma levels were considerably higher when compared to micronized isoxicam. A mean peak plasma concentration of 14.8±4.9 µg/ml was observed at four hours postdose for micronized isoxicam. Peak plasma concentrations for individual animals ranged from 9.92 to 19.0, 19.0 µg/ml between three and six hours postdose. Mean plasma concentrations proceeded to decline to the last sampling period of 240 hours where 0.82±0.3 µg/ml was observed for arginine isoxicamate but were not detectable for micronized isoxicam.

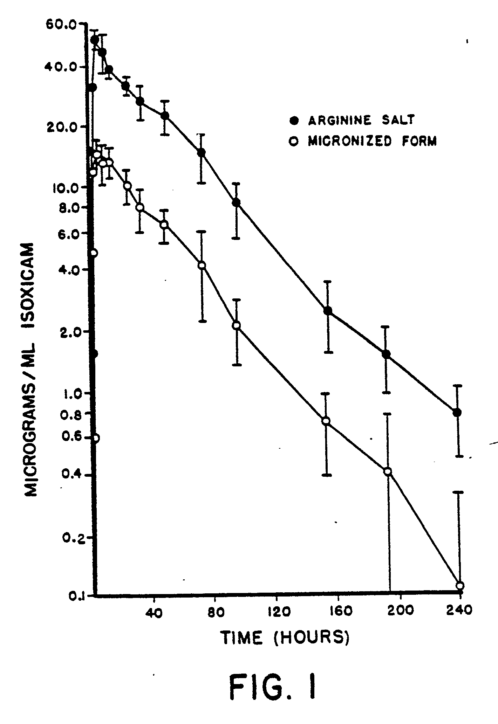

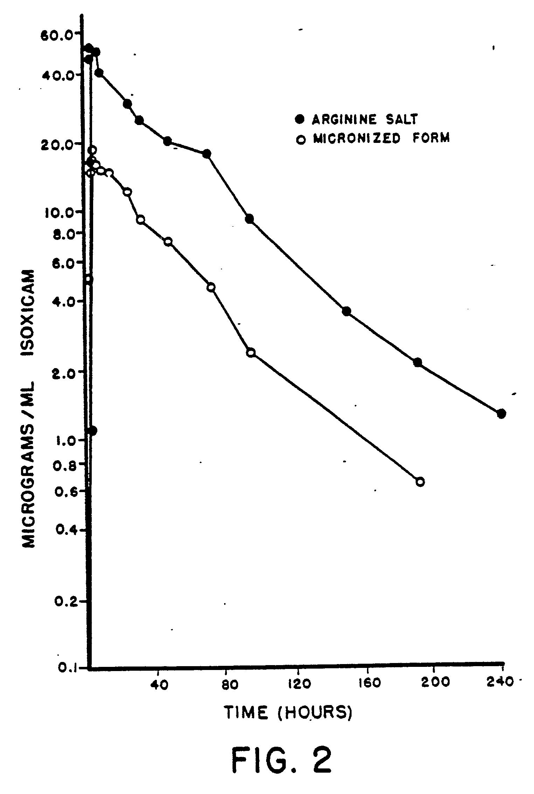

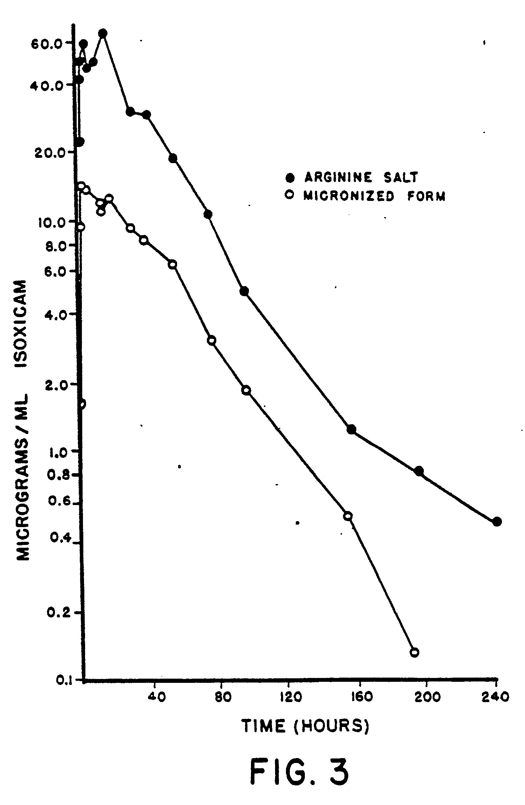

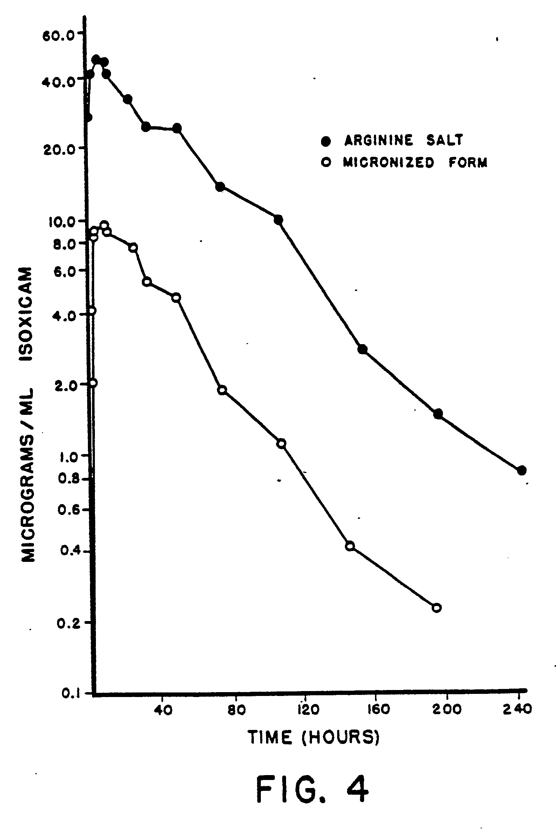

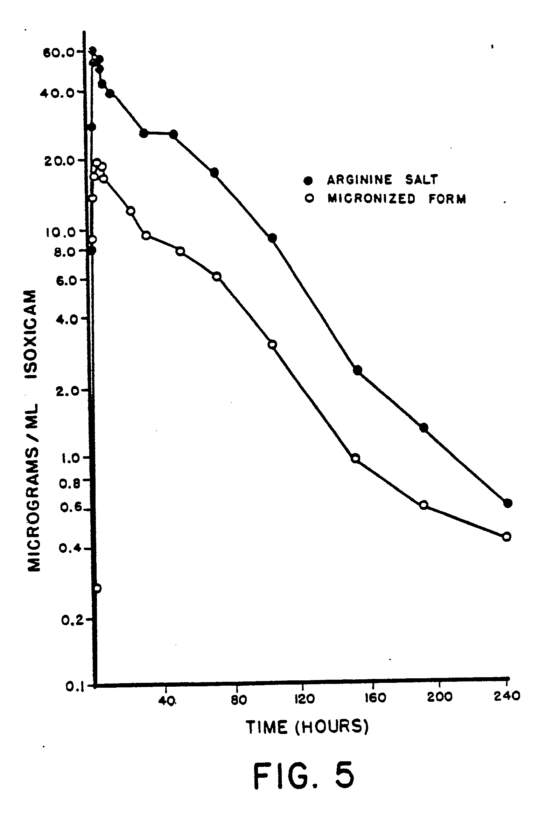

Calculated elimination half-lives using a linear regression analysis of the semilogarithmic data are shown in Table 2. Mean half-lives of 36.2±7.5 hours and 35.5±7.1 hours were found for arginine isoxicamate and micronized isoxicam. Mean Cmax values were dissimilar with a value of 56.2±5.6 µg/ml observed for arginine isoxicamate and 15.4±4.2 µg/ml for micronized isoxicam. A graphical presentation of the mean plasma concentration-time profile is shown in Figure 1. Individual animal data are presented in Figures 2—5.

Areas under the plasma concentrations versus time curve ($AUC^{0-\infty}$) were calculated using the trapezoidal rule and extrapolated to infinity with the appropriate elimination rate constants. In every case, the $AUC^{0-\infty}$ for arginine isoxicamate was greater than micronized isoxicam. The mean $AUC^{0-\infty}$ for arginine isoxicamate and isoxicam were 2840±170 µg · hr · ml$^{-1}$ and 808±230 µg · hr · ml$^{-1}$, respectively.

The relative bioavailability of L-arginine isoxicamate was determined from a comparison of the individual dog $AUC^{0-\infty}$ arginine salt/$AUC^{0-\infty}$ arginine salt/$AUC^{0-\infty}$ micronized isoxicam. The relative bioavailability in the four dogs was calculated to be 539, 278, 368, and 313. The mean relative bioavailability (arginine isoxicamate/micronized isoxicam) of arginine isoxicamate was determined to be 374 (±116)%. These data are summarized in Table 2.

## TABLE 1
### Plasma levels of isoxicam in dogs arginine salt versus micronized 100 mg/dog (Isoxicam equivalents) (µg/ml)

| Time | Animals dosed with arginine isoxicamate | | | | | | |
| | 1930 | 1942 | 117668 | 120064 | Mean | S.D. | RSD |
|---|---|---|---|---|---|---|---|
| Pre-Rx | N.D. | N.D. | N.D. | N.D. | — | — | — |
| 0.5 | 29.6 | 7.99 | 22.8 | 1.11 | 15.4 | 13.1 | 85.3 |
| 1.0 | 43.4 | 28.1 | 42.6 | 15.5 | 32.4 | 13.3 | 41.0 |
| 2.0 | 51.4 | 58.6 | — | 51.8 | 53.9 | 4.0 | 7.5 |
| 3.0 | 47.5 | 51.7 | 55.1 | 46.3 | 50.2 | 4.0 | 8.0 |
| 4.0 | 48.9 | 53.9 | 59.3 | 50.5 | 53.2 | 4.6 | 8.7 |
| 6.0 | 48.3 | 50.5 | 50.2 | 49.6 | 49.6 | 1.0 | 2.0 |
| 8.0 | 42.6 | 42.2 | 63.0 | 42.0 | 47.4 | 10.4 | 21.9 |
| 12.0 | 37.8 | 39.4 | — | — | 38.6 | 1.1 | 2.9 |
| 24.0 | 32.8 | 35.6 | 30.5 | 31.3 | 32.6 | 2.2 | 6.9 |
| 32.0 | 25.2 | 25.7 | 29.6 | 34.8 | 28.8 | 4.4 | 15.4 |
| 48.0 | 25.0 | 25.5 | 18.6 | 21.5 | 22.6 | 3.2 | 14.3 |
| 72.0 | 14.6 | 17.1 | 10.7 | 17.6 | 15.0 | 3.2 | 21.0 |
| 96.0 | 10.2 | 8.96 | 5.10 | 9.39 | 8.41 | 2.3 | 27.0 |
| 153.5 | 2.72 | 2.34 | 1.27 | 3.52 | 2.46 | 0.9 | 38.0 |
| 192.0 | 1.56 | 1.28 | 0.85* | 2.17* | 1.46 | 0.6 | 37.8 |
| 240.0 | 0.89 | 0.60 | 0.51 | 1.26 | 0.82 | 0.3 | 41.5 |

## EP 0 140 492 B1

TABLE 1 (continued)

| Time | Animals dosed with micronized isoxicam | | | | | | |
|---|---|---|---|---|---|---|---|
| | 1930 | 1942 | 117668 | 120064 | Mean | S.D. | RSD% |
| Pre-Rx | N.D. | N.D. | N.D. | N.D. | — | — | |
| 0.5 | 2.14 | 0.27 | N.D. | N.D. | 1.20 | 1.3 | 110.0 |
| 1.0 | 4.07 | 8.92 | 1.59 | 5.10 | 4.92 | 3.0 | 61.9 |
| 2.0 | 9.39 | 13.6 | 9.72 | 14.3 | 11.8 | 2.6 | 21.8 |
| 3.0 | 9.07 | 17.5 | 14.3 | 17.7 | 14.6 | 4.0 | 27.5 |
| 4.0 | 8.50 | 19.0 | 13.3 | 18.5 | 14.8 | 4.9 | 33.3 |
| 6.0 | 9.92 | 18.2 | 12.4 | 16.4 | 14.2 | 3.8 | 26.4 |
| 8.0 | 9.91 | 16.8 | 11.8 | 15.6 | 13.3 | 3.2 | 23.8 |
| 12.0 | — | — | 12.3 | 15.2 | 13.8 | 2.1 | 14.9 |
| 24.0 | 7.93 | 11.8 | 9.30 | 12.1 | 10.3 | 2.0 | 19.5 |
| 32.0 | 5.25 | 9.38 | 8.18 | 9.13 | 7.98 | 1.9 | 23.7 |
| 48.0 | 4.77 | 7.92 | 6.46 | 7.63 | 6.70 | 1.4 | 21.4 |
| 72.0 | 2.72 | 6.04 | 3.04 | 4.60 | 4.10 | 1.5 | 37.4 |
| 96.0 | 1.17 | 3.02 | 1.80 | 2.44 | 2.11 | 0.8 | 37.9 |
| 153.5 | 0.41 | 0.94 | 0.52 | 0.92 | 0.70 | 0.3 | 39.0 |
| 192.0 | 0.22* | 0.60* | 0.13 | 0.66 | 0.40 | 0.3 | 66.2 |
| 240.0 | N.D. | 0.42 | N.D. | N.D. | 0.10 | 0.2 | 200.0 |

*Actual time=193 hours

4

TABLE 2
Bioavailability parameters for aginine isoxicamate and micronized
isoxicamate after oral administration of 100 mg
(free acid equivalents) to beagle dogs

| Dog number | $AUC^{0-\infty}$ $\mu g \times hr \times ml^{-1}$ | | Relative Bioavailability %(Arg/isoxicam) | Half-life (hours) | | Tmax (hours) | | Cmax ($\mu g/ml$) | |
|---|---|---|---|---|---|---|---|---|---|
| | arginine | isoxicam | | Arginine | Isoxicam | Arginine | Isoxicam | Arginine | Isoxicam |
| 1930 | 2910 | 540 | 539 | 38.8 | 32.6 | 2 | 6 | 51.4 | 9.92 |
| 1942 | 2920 | 1050 | 278 | 34.4 | 42.4 | 2 | 4 | 58.6 | 19.0 |
| 117668 | 2590 | 703 | 368 | 27.0 | 26.8 | 6 | 3 | 59.3 | 14.3 |
| 120064 | 2950 | 941 | 313 | 44.8 | 40.1 | 2 | 4 | 51.8 | 18.5 |
| Mean | 2840 | 808 | 374 | 36.2 | 35.5 | 3 | 4.2 | 55.3 | 15.4 |
| SD | 170 | 230 | 116 | 7.5 | 7.1 | 2 | 1.3 | 4.3 | 4.2 |
| RSD% | 6.0 | 28.5 | 30.9 | 20.7 | 20.1 | 66.7 | 29.6 | 7.7 | 27.4 |

Generally speaking, the L-arginine salt of isoxicam is indicated in conditions such as pain resulting from, for example, arthritis or bursitis. A daily dosage regimen of half the amount of the free acid, i.e., about 0.25 g to about 1 g in several divided doses is recommended for a mammal weighing about 70 kg body weight to relieve the pain and swelling associated with these conditions.

The following Examples are illustrative of the invention.

Example 1

L-(+)-Arginine, 5.23 g, 0.03 moles, was dissolved in 80 ml of water in a 500 ml round bottom flask. Isoxicam, 10.05 g, 0.03 moles, and 150 ml of methanol were added and the mixture refluxed for two days. The resulting mixture was filtered through a medium-pore sintered glass disc and the filtrate evaporated to dryness. The solid residue was recrystallized from methanol to give 13.2 g (86.4%) of a fine, pale yellow crystalline powder, mp 220°C with decomposition.

Analysis calcd. for $C_{20}H_{27}N_7O_7S$:

C, 47.14; H. 5,34; N, 19.24; S. 6.29.

Found; C, 46.84; H, 5.26; N, 19.12, S. 6.45.

Solubility: 19 mg per ml in water; pH of saturated solution 8.8.

Example 2

A tablet is formed from the following ingredients:

|  | mg/tablet |
|---|---|
| Arginine isoxicamate, 304 mg equivalent to isoxicam | 200.0 |
| Microcrystalline cellulose | 80.0 |
| Lactose | 16.0 |
| Corn starch | 80.0 |
| Hydroxypropyl cellulose | 15.0 |
| Calcium stearate | 5.0 |

Hydrate the hydroxypropyl cellulose in about 0.15 ml of water. Blend the arginine isoxicamate, microcrystalline cellulose, lactose, corn starch and mix with the hydroxypropyl cellulose solution to make a moist granulation. Dry granulation at 50°C, add the calcium stearate, mix and blend well.

Compress into tablets weighing 500 mg, on a suitable tablet compressing machine.

Example 3

A two piece hard shell capsule is prepared from the following ingredients:

|  | mg/capsule |
|---|---|
| Arginine isoxicamate, 152 mg equivalent to Isoxicam | 100.0 |
| Microcrystalline cellulose | 105.0 |
| Corn starch | 100.0 |
| Magnesium stearate | 3.0 |

All the above ingredients are blended together until homogeneous. The resultant mixture is filled into a Number 1 two piece hard gelatin capsule at fill weight of 360 mg, using a conventional capsule filling machine.

Example 4
A syrup formulation was prepared from the following ingredients:

|  | 5 ml dose contains |
| --- | --- |
| Arginine isoxicamate, 76 mg equivalent to isoxicam | 50.0 mg |
| Sugar, granulated | 2.5 g |
| Glucose, liquid | 1.1 g |
| Alcohol, USP | 0.05 ml |
| Oil of orange | 0.5 µl |
| Peach flavor imitation | 0.06 µl |
| Menthol | 0.25 mg |
| Water, sufficient quantity to make | 5.0 ml |

Dissolve the arginine isoxicamate in 1.5 ml water, heat to 60°C, add and dissolve the sugar with stirring. Add the glucose, mix until clear solution results. Dissolve the oil of orange, peach flavor, and menthol in the alcohol. Add flavor solution to the aqueous drug solution, slowly and with rapid stirring. Filter the syrup until clear and make to final volume with addition of water and mix.

Example 5
An injectable solution was prepared from the following ingredients:

|  | mg/l ml |
| --- | --- |
| Arginine isoxicamate, 15.2 mg equivalent to Isoxicam | 10.0 |
| Water for injection sufficient quantity to make | 1.0 ml |

Dissolve the arginine isoxicamate in the water, check pH (8.7—8.9). Adjust and recheck final volume to 1.0 ml. Sterilize the solution by filtration through a sterile 0.2 µm membrane filter into a sterile receiving vessel.

Aseptically fill the desired volume into previously sterilized ampuls or other previously sterilized containers. Close and seal the containers.

**Claims for the Contracting States: BE, CH, DE, FR, GB, IT, LI, LU, NL, SE**

1. A compound having the name L-arginine isoxicamate.
2. A compound according to Claim 1, and which has 1:1 stoichiometry.
3. A compound according to Claim 2, which is in an anhydrous, crystalline state.
4. A pharmaceutical composition, suitable for use as an anti-inflammatory agent or as an analgesic, comprising an effective amount of a compound as claimed in Claim 1, 2 or 3 in admixture with a pharmaceutically acceptable carrier.
5. A composition as claimed in Claim 4, in oral dosage form.
6. A process for preparing L-arginine isoxicamate, which process comprises heating L-arginine and isoxicam together in a solvent.
7. A process according to Claim 6, wherein the solvent is aqueous methanol.
8. A process according to Claim 6 or 7, wherein the heating is carried out at the boiling point of the solvent.
9. A process for preparing a pharmaceutical composition, which process comprises admixing an effective amount of a compound as claimed in Claim 1, 2 or 3 with a pharmaceutically acceptable carrier.
10. For use as an anti-inflammatory agent, a compound as claimed in any one of Claims 1 to 3 or a pharmaceutical composition as claimed in Claim 4.

7

**EP 0 140 492 B1**

11. For use as an anti-inflammatory agent, a compound as claimed in any one of Claims 1 to 3 or a pharmaceutical composition as claimed in Claim 4, in oral dosage form.

**Claims for the Contracting State: AT**

1. A process for preparing a compound having the name L-arginine isoxicamate, which process comprises heating L-arginine and isoxicam together in a solvent.

2. A process according to Claim 1, wherein the solvent is aqueous methanol.

3. A process according to Claim 1 or 2, wherein the heating is carried out at the boiling point of the solvent.

4. A process according to Claim 1, 2 or 3, for preparing L-arginine isoxicamate having 1:1 stoichiometry.

5. A process according to Claim 4, wherein the L-arginine isoxicamate prepared as in an anhydrous, crystalline state.

6. A process for preparing a pharmaceutical composition which is suitable for use as an anti-inflammatory agent or as an analgesic, which process comprises admixing an effective amount of L-arginine isoxicamate with a pharmaceutical carrier.

7. A process according to Claim 6, wherein the composition is in oral dosage form.

8. For use as an anti-inflammatory agent, a compound prepared by a process as claimed in any one of Claims 1 to 5 or a pharmaceutical composition prepared by a process as claimed in Claim 6.

9. For use as an anti-inflammatory agent, a compound prepared by a process as claimed in any one of Claims 1 to 5 or a pharmaceutical composition prepared by a process as claimed in Claim 6, in oral dosage form.

**Patentansprüche für die Vertragsstaaten: BE, CH, DE, FR, GB, IT, LI, LU, NL, SE**

1. Verbindung mit dem Namen L-Arginin-Isoxicamat.

2. Verbindung nach Anspruch 1, die im stöchiometrischen Verhältnis von 1:1 vorliegt.

3. Verbindung nach Anspruch 2, welche sich in einem wasserfreien kristallinen Zustand befindet.

4. Pharmazeutische Zusammensetzung, geeignet zur Verwendung als entzüdungshemmendes Mittel oder als Analgetikum, umfassend eine wirksame Menge einer Verbindung, wie im Anspruch 1, 2 oder 3 beansprucht, in Mischung mit einem pharmazeutisch akzeptablen Träger.

5. Zusammensetzung, wie im Anspruch 4 beansprucht, in oraler Dosierungsform.

6. Verfahren zur Herstellung von L-Arginin-Isoxicamat, welches Verfahren das gemeinsame Erhitzen von L-Arginin und Isoxicam in einem Lösungsmittel umfaßt.

7. Verfahren nach Anspruch 6, worin das Lösungsmittel wässeriges Methanol ist.

8. Verfahren nach Anspruch 6 oder 7, worin das Erhitzen am Siedepunkt des Lösungsmittels durchgeführt wird.

9. Verfahren zur Herstellung einer pharmazeutischen Zusammensetzung, welches Verfahren das Mischen einer wirksamen Menge einer Verbindung, wie im Anspruch 1, 2 oder 3 beansprucht, mit einem pharmazeutisch akzeptablen Träger umfaßt.

10. Verwendung einer Verbindung, wie in irgendeinem der Ansprüche 1 bis 3 beansprucht, oder einer pharmazeutischen Zusammensetzung, wie in Anspruch 4 beansprucht, als entzündungshemmendes Mittel.

11. Verwendung einer Verbindung, wie in irgendeinem der Ansprüche 1 bis 3 oder einer pharmazeutischen Zusammensetzung, wie im Anspruch 4 beansprucht, in oraler Dosierungsform als entzündungshemmendes Mittel.

**Patentansprüche für den Vertragsstaat: AT**

1. Verfahren zur Herstellung einer Verbindung mit dem Namen L-Arginin-Isoxicamat, welches Verfahren das gemeinsame Erhitzen von L-Arginin und Isoxicam in einem Lösungsmittel umfaßt.

2. Verfahren nach Anspruch 1, worin das Lösungsmittel wässeriges Äthanol ist.

3. Verfahren nach Anspruch 1 oder 2, worin das Erhitzen am Siedepunkt des Lösungsmittels durchgeführt wird.

4. Verfahren nach Anspruch 1, 2 oder 3 zur Herstellung von L-Arginin-Isoxicamat, welches im stöchiometrischen Verhältnis von 1:1 vorliegt.

5. Verfahren nach Anspruch 4, worin das hergestellte L-Arginin-Isoxicamat sich in einem wasserfreien kristallinen Zustand befindet.

6. Verfahren zur Herstellung einer pharmazeutischen Zusammensetzung, welches zur Verwendung als entzündungshemmendes Mittel oder als Analgetikum geeignet ist, welches Verfahren das Mischen einer wirksamen Menge von L-Arginin-Isoxicamat mit einem pharmazeutischen Träger umfaßt.

7. Verfahren nach Anspruch 6, worin die Zusammensetzung orale Dosierungsform aufweist.

8. Verwendung einer Verbindung, welche mittels eines, wie in irgendeinem der Ansprüche 1 bis 5 beanspruchten Verfahrens, hergestellt wurde, oder einer pharmazeutischen Zusammensetzung, welche mittels eines wie im Anspruch 6 beanspruchten Verfahrens hergestellt wurde, als entzündungshemmendes Mittel.

9. Verwendung einer Verbindung, welche mittels eines, wie in irgendeinem der Ansprüche 1 bis 5 beanspruchten Verfahrens hergestellt wurde, oder einer pharmazeutischen Zusammensetzung, welche mittels eines wie im Anspruch 6 beanspruchten Verfahrens hergestellt wurde, in oraler Dosierungsform als entzündungshemmendes Mittel.

**Revendications pour les Etats Contractants: BE, CH, DE, FR, GB, IT, LI, LU, NL, SE**

1. Un dérivé portant le nom d'isoxicamate de L-arginine.

2. Un dérivé selon la revendication 1, présentant une stoechiométrie 1/1.

3. Un dérivé selon la revendication 2, qui se trouve à l'état anhydre cristallin.

4. Une composition pharmaceutique convenant à l'utilisation comme agent anti-inflammatoire ou comme analgésique, comprenant une quantité efficace d'un dérivé selon la revendication 1, 2 ou 3, en mélange avec un support pharmaceutiquement acceptable.

5. Une composition selon la revendication 4, sous forme de dosage par voie orale.

6. Un procédé de préparation d'isoxicamate de L-arginine, ce procédé consistant à chauffer de la L-arginine et de l'isoxicame ensemble dans un solvant.

7. Un procédé selon la revendication 6, dans lequel le solvant est le méthanol aqueux.

8. Un procédé selon la revendication 6 ou 7, dans lequel on met le chauffage en oeuvre au point d'ébullition du solvant.

9. Un procédé de préparation d'une composition pharmaceutique consistant à mélanger une quantité efficace d'un dérivé selon la revendication 1, 2 ou 3, avec un support pharmaceutiquement acceptable.

10. Pour l'utilisation comme agent anti-inflammatoire, un dérivé selon l'une quelconque des revendications 1 à 3, ou une composition pharmaceutique selon la revendication 4.

11. Pour l'utilisation comme agent anti-inflammatoire, un dérivé selon l'une quelconque des revendications 1 à 3, ou une composition pharmaceutique selon la revendication 4, sous forme de dosage oral.

**Revendications pour l'Etat Contractant: AT**

1. Un procédé de préparation d'un dérivé portant le nom d'isoxicamate de L-arginine, consistant à chauffer ensemble dans un solvant de la L-arginine et de l'isoxicame.

2. Un procédé selon la revendication 1, dans lequel le solvant est le méthanol aqueux.

3. Un procédé selon la revendication 1, dans lequel on met le chauffage en oeuvre au point d'ébullition du solvant.

4. Un procédé selon la revendication 1, 2 ou 3, de préparation de l'isoxicamate de L-arginine présentant une stoechiométrie 1/1.

5. Un procédé selon la revendication 4, dans lequel l'isoxcamate de L-arginine préparé se trouve à l'état cristallin anhydre.

6. Un procédé de préparation d'une composition pharmaceutique convenant à l'utilisation comme agent anti-inflammatoire ou comme analgésique, ce procédé consistant à mélanger une quantité efficace d'isoxicamate de L-arginine avec un support pharmaceutique.

7. Un procédé selon la revendication 6, dans lequel la composition se trouve sous forme de dosage par voie orale.

8. Pour l'utilisation comme agent anti-inflammatoire, un dérivé préparé par un procédé selon l'une quelconque des revendications 1 à 5, ou une composition pharmaceutique préparée par un procédé selon la revendication 6.

9. Pour l'utilisation comme agent anti-inflammatoire, un dérivé préparé par un procédé selon l'une quelconque des revendications 1 à 5, ou une composition pharmaceutique préparée par un procédé selon la revendication 6 sous forme de dosage par voie orale.

FIG. 1

FIG. 2

FIG. 3

FIG. 4

4

FIG. 5